# EUROPEAN PATENT SPECIFICATION

(11) **EP 4 285 859 B1**
(45) Date of publication and mention of the grant of the patent: **15.10.2025**
(21) Application number: 23169144.5
(22) Date of filing: 21.04.2023
(51) Int. Cl.: A61B 90/40, A61B 90/70, A61L 2/14, H05H 1/24

(54) **SYSTEM FOR ERADICATION OF PATHOGENIC MICROORGANISMS FROM FLAT SURFACES OR SKIN TISSUE**
SYSTEM ZUR BESEITIGUNG PATHOGENER MIKROORGANISMEN AUF EBENEN OBERFLÄCHEN ODER HAUTGEWEBE
SYSTÈME D'ÉRADICATION DE MICRO-ORGANISMES PATHOGÈNES DE SURFACES PLANES OU DE TISSUS CUTANÉS

(30) Priority: 02.06.2022 PL 44135622
(43) Date of publication of application: 06.12.2023
(73) Proprietor: Uniwersytet Gdanski, 80-309 Gdansk (PL); Politechnika Wroclawska, 50-370 Wroclaw (PL)
(72) Inventor: Motyka-Pomagruk, Agata, 80-041 Gdank (PL); Sledz, Wojciech, 10-123 Olsztyn (PL); Prusinski, Michal, 81-611 Gdynia (PL); Orlowski, Jakub, 76-004 Sianow (PL); Babinska, Weronika, 80-766 Gdansk (PL); Lojkowska, Ewa, 80-041 Gdansk (PL); Dzimitrowicz, Anna, 52-117 Wroclaw (PL); Jamroz, Piotr, 52-117 Wroclaw (PL); Pohl, Pawel, 51-689 Wroclaw (PL); Dora, Jerzy, 51-361 Wilczyce (PL)
(74) Representative: Czarnik, Maciej

(56) References cited:
- US-A1- 2010 296 977
- US-A1- 2012 064 016
- US-A1- 2020 155 266
- US-A1- 2020 316 239

## Description

The invention refers to method for inactivation of microorganisms, for instance belonging to the genera *Staphylococcus*, *Candida* or *Dermatophilus*, which are responsible for infections in animals and humans, skin infections in particular, by subjection to a direct exposure of a flat surface, especially surfaces found in hospital environments or exemplary, but not claimed, skin tissue, to the selected type of atmospheric plasma.

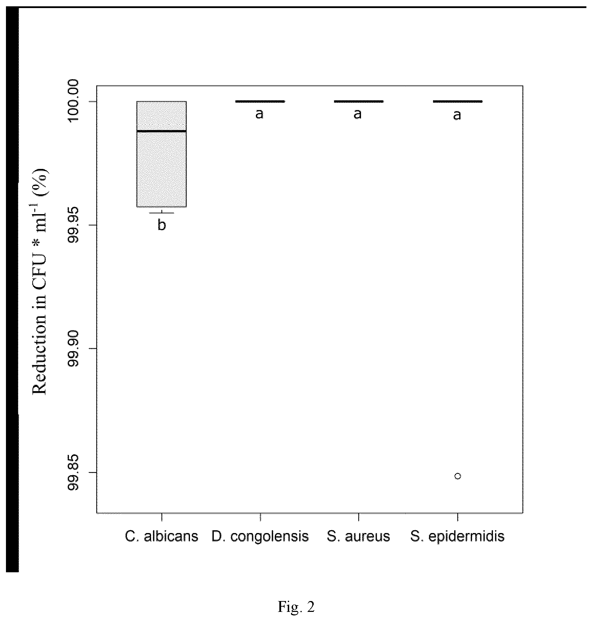

In the publication by Sedghizadeh, P. P., Chen, M. T., Schaudinn, C., Gorur, A., & Jiang, C. entitled "Inactivation kinetics study of an atmospheric-pressure cold-plasma jet against pathogenic microorganisms" (IEEE Transactions on Plasma Science 2012, 40(11): 2879) the Cold Atmospheric Pressure Plasma (CAPP) was used to inhibit proliferation of pathogenic microorganisms such as *Staphylococcus aureus*, *Staphylococcus epidermidis*, *Pseudomonas aeruginosa*, *Escherichia coli* and *Candida albicans.* Plasma was initiated in a stationary plasma system, in a helium atmosphere, by utilizing pulsed discharges (1-2 kHz) in a form of a plasma jet to initiate CAPP. The system used for elimination of microorganisms comprised of a nanosecond high voltage (6 kV) radio frequencies (1-2 kHz) generator and a CAPP tip. Agar plates previously inoculated with 10⁵, 10⁶, 10⁷ cells/cm² of the analyzed microorganisms were exposed for 180s to the so-generated CAP. After the microorganisms were treated with CAPP, a centrally located zone of 12 mm in diameter was determined within the microorganisms-inoculated agar plates, which was called by the authors region of interest (ROI). ROI zones were cut from the agar plates and suspended in sterile water as a result of the sonication process conducted at room temperature for 5 min. Subsequently, bacterial suspensions from the sonicated ROI zones were serially diluted and plated on agar plates prior to 16h incubation, counting of colony-forming units and subsequent calculation of logarithms of reduction in the number of CFUs of pathogenic microorganisms. Based on the analyses designed in this way, the effectiveness of the CAPP application, initiated for 180 s, against pathogenic bacteria was reported to exceed 4 logarithms of reduction in the number of colony-forming units, while in the case of the included yeast *C*. *albicans,* the effectiveness of the described method exceeded 3.5 logs. The authors also found that shorter exposure times to the initiated in this way CAP revealed differences in the susceptibility of the analyzed microorganisms to the applied therapy. 5-30 s exposure to the utilized plasma jet showed a higher susceptibility of Gram(+) bacteria to shorter exposure times compared to Gram(-) bacteria. The opposite effect was observed during longer CAPP operation times, *e.g.* 180 s. However, the research methodology utilized in this work was not conventional and fully compliant with the standards of microbiological work. The agar plates containing culture media are used routinely to multiply microorganisms under laboratory conditions, therefore they are not a properly selected material for CAPP exposure. Such experimental planning does not mimic the real-life situation oriented towards application of CAPP against pathogenic microorganisms. The presented data also provide no information on effectiveness of eradication of biofilm-forming or host tissue-colonizing microorganisms. In addition, the number of microbial cells, according to the literature conventions, is presented either directly as a number of CFUs or as a number of colony-forming units suspended in a stated volume of a solution (CFU/cm³). In the cited paper, the number of microbial cells per surface units (cells/cm²) was reported, however, this manuscript lacks a description of how this parameter had been calculated. Thus, it is uncertain whether ROIs were cut from agar plates inoculated with the tested microorganisms, which included either plasma-treated, non-plasma-treated or subjected solely to the discharge gas samples, to estimate this value, or in which way the assumptions were made on the number of cells in a given experiment. The issue of how to count the cells seems to be crucial, especially since in section B it was described that this value was used to estimate the logarithms of reduction in the number of CFUs of pathogenic microorganisms, *i.e.* de facto to assess the effectiveness of the entire process. Not without significance is the fact that the authors reported interdependence between the effectiveness of microorganisms' eradication and the number of pathogenic cells suspended in the solution (section III). Another aspect requiring higher attention is the issue of determining the diameter of the ROI zones. It was not specified on what basis it was assumed that a zone of a diameter 10 times larger than the diameter of the plasma jet tip should be analyzed. Considering the diameters of the microbial growth inhibition zones presented in Figure 2 of the manuscript, designation of the ROI zones by the authors seems to be quite random.

In another manuscript by Nasir, N. M., Lee, B. K., Yap, S. S., Thong, K. L., & Yap, S. L. entitled "Cold plasma inactivation of chronic wound bacteria" (Archives of Biochemistry and Biophysics 2016, 605: 76) the use of CAPP, which was this time generated in two alternative systems, *i.e.* generated in the form of a plasma jet in a planar plasma system with a Dielectric Barrier Discharge (DBD) plasma, as well as in a capillary-guided (CGCD) portable corona discharge plasma system, was described against bacteria responsible for acute and chronic wound infections belonging to the species *S*. *aureus* and *P. aeruginosa.* Clinical isolates of *S*. *aureus* (three isolates) and *P. aeruginosa* (four isolates) were included in these analyses. The CAPP system applied to inactivate bacterial cells worked with the use of DBD. This system comprised steel electrodes of 6.0 cm in diameter and of 1.5 cm in thickness. As an electrical barrier Pyrex glass 2.0 thick was used. 50 µl of the bacterial overnight culture was placed on a glass slide, which was then exposed for up to 60 min to CAPP generated in a stationary plasma system, as a result of DBD initiation in the space between the electrodes made of stainless steel (diameter 6.0 cm, thickness 1.5 cm). Subsequently, the glass slides containing microorganisms exposed to the applied discharges were placed in 50 ml of phosphate buffered sodium chloride (PBS) and vortexed. Afterwards, the resultant bacterial suspensions were serially diluted and plated on TSA media. The plates were incubated at 37°C for 24 h in order to count CFUs/ml. The exposure of *S*. *aureus* strains to DBD for a period of 60 minutes resulted in the reduction in the number of colony-forming units of these pathogenic microorganisms that exceeded 2 logs. This type of discharge turned out to be much more efficient against four strains of *P. aeruginosa*, as the reduction in the number of CFU/ml exceeded 9 logarithms after 40 mins of treatment. On the other hand, the use of CGCD resulted in the reduction in the number of CFU/ml > 9 logarithms already post 10 mins or 30 mins of plasma exposure, regarding *P. aeruginosa* or *S*. *aureus,* respectively. Although the reported results seem to be unconvincing. According to the available information, Figure 14 of the manuscript compares the effectiveness of DBD and CGCD against *P. aeurginosa* strain named pa 20, presenting in a different manner data already included in Figures 10 and 12. In the case of CGCD the depicted data seems convergent, however the dynamics of the decrease in the viability of the strain pa 20 due to DBD exposure as visualized in Figure 10, does not appear to coincide with the data previously shown in Figure 14. In section 2.3, there is information that CAPP was applied from 1 min to 30 mins, though the charts included in the work and the "results" section contain data from the exposure time up to 60 mins. Therefore, apart from relatively long exposure times applied, there is no certainty concerning the effectiveness of such approach.

In the next work, this time published by Maisch, T., Shimizu, T., Li, Y. F., Heinlin, J., Karrer, S., Morfill, G., & Zimmermann, J. L. under the title "Decolonisation of MRSA, S. aureus and E. coli by cold-atmospheric plasma using a porcine skin model in vitro " (PloS one 2012 277(4):e34610) direct use of CAPP was reported for the first time to induce decolonization of Gram(+) and Gram(-) bacteria, which are pathogenic to humans and animals, from a porcine skin model. The effectiveness of two commercially available CAPP-generating devices, *i.e.* FlatPlaSter and miniFlatPlaSter, was tested in terms of their ability to eradicate three *Staphylococcus aureus* strains (two methicillin-resistant: ATCC BAA-44 and ATCC 43300 in addition to one methicillin-sensitive strain: ATCC 25923) and one strain of *Escherichia coli* ATCC 25922. The CAPP system was described, which was used to eradicate bacteria, as built of flat electrodes made of brass and steel mesh, enclosed in a plastic container. A voltage of 9 kV and a frequency of 1 kHz was used to generate CAPP. The application of the FlatPlaSter device for 6 minutes led to a 3-log reduction in the number of colony-forming units (CFUs) of *S*. *aureus* and *E*. *coli* strains. Extending the exposure time to CAPP to 8 mins, resulted in an increase in the efficiency of the applied device as the reduction in the number of CFUs was higher than 5 logarithms. Similar potency of the CAPP-generating device was found for both methicillin-resistant and methicillin-sensitive strains of *Staphylococcus aureus.* Compared to FlatPlaSter, the miniFlatPlaSter system turned out to be more efficient in combating the analyzed microorganisms, as already 5 mins subjection to CAPP operated in this way led to the reduction in the number of CFUs over 3 logs. There was also reported an absence of damage to the CAPP-treated skin tissue. It is worth emphasizing though that the CAPP was applied immediately after initiation of bacterial infection (inoculum: 10⁶⁻⁷ CFU/ml) on the selected porcine skin model. By applying this methodology, microorganisms are unable to colonize the skin tissue, which means that removal of these cells from the CAP-exposed surface was significantly facilitated. The selected experimental model does not reliably reflect the real necessity for eradication of microorganisms exhibited by the medical sector as in the latter case the microbes would have already colonized a host tissue and/or created a biofilm layer impeding drugs penetration.

Additional relevant prior art is disclosed in US 2020/155266 A1, US 2020/316239 A1 and US 2012/064016 A1.

The difficult-to-heal wounds are a source of acute and/or chronic pain, which notably contributes to lowering the quality of life and leads to poorer prognosis for farm animals infected with multidrug-resistant bacterial strains. The most often isolated from animal wounds bacterial strains belong to the species that also infect human patients in the hospital setting. In this view, the elevated virulence in addition to difficulties related to combating pathogens obtained from the hospital facilities, as well as the limited potency of conventional antibiotic therapies should be emphasized. Currently, antibiotics are very often prescribed to combat diseases caused by viruses and the drug resistance profiles of the treated bacteria are not routinely determined. The broad-spectrum drugs are commonly prescribed in wrong doses and the patients discontinue administration of the medication as soon as they feel better. For ubiquitous presence of these drugs in the natural environment, it is significant that antibiotics are used excessively in animal husbandry to boost biomass gain and for preventive purposes. The high availability of antibiotic molecules in the natural environment results in establishment of selective pressure towards microorganisms exposed to their sublethal doses, which leads to the spread and maintenance of multidrug resistance genetic determinants among bacterial populations. Considering that the therapeutic options used so far are highly insufficient, the aim behind this invention was to develop new, ecological as well as effective methods of combating pathogens responsible for infections in animals and humans, in particular skin infections.

The current state of the technology indicates that only partial attempts have been made to combat the microorganisms responsible for wound infections with CAPP, as described in the above-mentioned literature reports. Although there is no certainty whether the presented results of scientific research shall be implemented with the expected outcomes, in a manner that does not require further experimental studies.

For this reason, effective methods for eradication of microorganisms responsible for skin infections, especially wounds, are sought, which was the main goal of this invention as defined by appended independent claims 1 and 7. Further embodiments are disclosed in the dependent claims. Methods relating to the application of the method of claim 1 on the body are included for illustrative purposes only and are not claimed.

The invention is system and method for combating microorganisms responsible for skin infections, especially wounds, from flat surfaces - skin, furniture surfaces, especially in hospitals, using direct exposure to CAPP, initiated as a result of generating DBD discharges in the form of a plasma jet.

The subject matter of the invention is method of combating microorganisms responsible for wound infections, characterized in that from flat surfaces, especially surfaces found in the hospital settings or skin tissues of animals and humans, by a direct exposure to CAPP, generated as a result of DBD initiation from a plasma jet (under helium atmosphere) in the developed plasma system, cells of pathogenic microorganisms are eliminated. The discharge system is also a subject of protection. The reaction-discharge system, or plasma system, is characterized in that it is built of the most important elements, *i.e*. a radio frequency generator, modulated by pulses, connected to: a) a quartz capillary with tungsten electrodes placed outside the quartz tube b) a cavity (tip) made of epoxy resin c) cavity filling of silica and an insulator, where helium is used as the discharge gas to ignite CAPP, the gas is injected into the reaction-discharge system at a flow rate of 2-10 L/min, and an alternating current is used to initiate the CAPP with a duty cycle of 70% and a frequency of modulation of 70%.

In this research, important conditions for generating CAPP were also determined, which contributed to increasing the effectiveness of the proposed method of eradication of microorganisms responsible for wound infections. The conducted analyses revealed a strong dependence of the implemented method on the conditions of generating CAPP, *i.e.* the duty cycle of 70%, the frequency of modulation of this current of 70%, the discharge gas flow rate in the range from 2 to 10 L/min, the temperature of the plasma cone <38 ° C, the time of exposure to CAPP of the treated surface in the range of 10 seconds to 10 minutes, preferably 2 mins, the number of single exposures of the treated material to CAPP in the range of 1-2, the distance between the exposed flat surface and the CAPP cone, *i.e.* from 2 mm to 10 cm , most preferably 3 cm. The selection of the above-listed parameters, especially the exposure time, depends on the microbial load, as well as the type and size of the sterilized surface.

Preferably, eradication of microorganisms responsible for wound infections is carried out in a plasma system, consisting of a current generator connected to: a) a quartz capillary with tungsten electrodes, b) a cavity made of E-57 epoxy resin, c) filling of the cavity made of silica and an insulator, *i.e. e.g.* ceramics.

Preferably, the method of eradicating microorganisms responsible for wound infections is carried out by treatment of the exposed surface with a DBD discharge once or twice, stating that one exposure lasts from 10 seconds to 10 mins, and most preferably 2 mins.

Preferably, the method of eradicating microorganisms responsible for wound infections is carried out at a distance between the exposed flat surface and the CAPP cone equaling 2 mm - 10 cm, most preferably 3 cm.

Preferably, the method of eradicating microorganisms responsible for wound infections is carried out by exposing cells of pathogenic microorganisms residing on the skin of humans and animals, in particular microorganisms from the species *S*. *aureus*, *S. epidermidis*, *C. albicans* and *Dermatophilus congolensis*, to the DBD discharge.

Preferably, the method of eradicating microorganisms responsible for wound infections is carried out by eliminating microorganisms that form a difficult-to-remove biofilm structure.

Preferably, the method of eradicating microorganisms responsible for wound infections is carried out without causing a temperature shock and visible damage to the flat surface treated with CAPP.

The subject of the invention is presented in a form of three examples illustrating the method of eradicating microorganisms responsible for wound infections by direct exposure to CAPP, generated as a result of initiation of DBD in the form of a plasma jet, and in drawing:

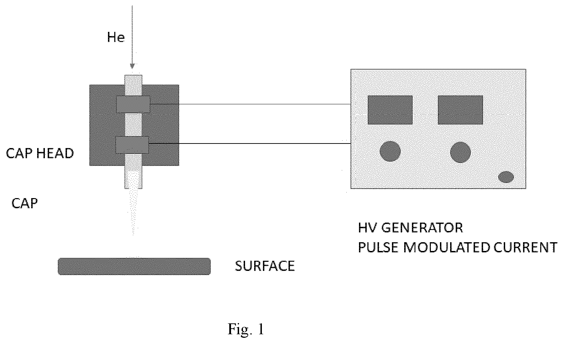

- Figure 1 shows a scheme of the reaction-discharge system for production of CAPP, generated as a result of DBD initiation from a plasma jet in a helium atmosphere, applied, according to the invention, to eliminate cells of pathogenic microorganisms of humans and animals. This reaction-discharge system, according to the invention, consists of a radio-frequency generator, modulated by pulses, connected to: a) a quartz capillary containing tungsten electrodes placed outside the quartz tube b) a cavity (tip) made of epoxy resin c) a filling of the cavity of silica and insulator, assuming also usage of helium as the discharge gas to produce CAPP, which is introduced into the reaction discharge system at a flow rate of 2-10 L min^{-1.}
- Figure 2 shows eradication, as a result of exposure to CAPP generated in a plasma system with DBD in the form of a plasma jet, of microorganisms belonging to the species *S*. *aureus, S. epidermidis, C. albicans* and *D. congolensis,* causing wound infections, which had been previously applied to the flat surface in a suspension of 0.5 McF optical density. The outcomes are depicted in a form of boxplot visualizing the median and individual data distribution quartiles of the observed percentage reduction in the number of colony-forming units of *S*. *aureus* ATCC 25923, *S*. *epidermidis* ATCC 14990, *C*. *albicans* ATCC 90028 and *D. congolensis* ATCC 14637, resulting of CAPP application according to the invention to the research samples in contrast to control samples that have not been treated with this discharge. The experiment was performed in three biological repeats, each of them included two technical replicates.

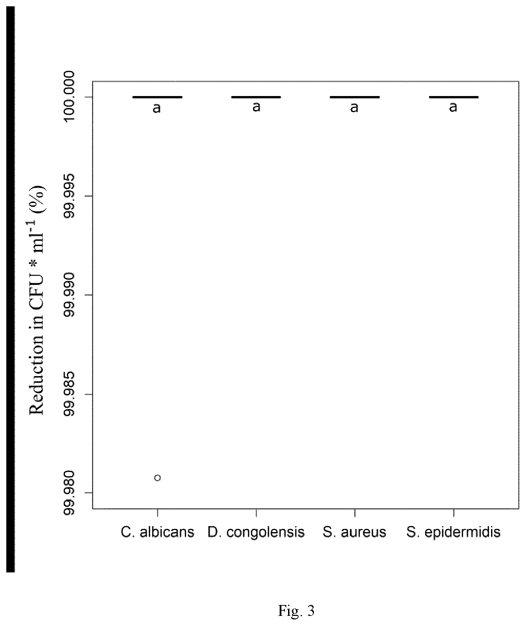

- Figure 3 shows eradication, as a result of exposure to CAPP generated in a plasma system with DBD in the form of a plasma jet, of microorganisms belonging to the species *S*. *aureus, S. epidermidis, C. albicans* and *D. congolensis,* causing wound infections, which had been previously applied to the flat surface in a suspension of 0.05 McF optical density. The outcomes are depicted in a form of boxplot visualizing the median and individual data distribution quartiles of the observed percentage reduction in the number of colony-forming units of *S*. *aureus* ATCC 25923, *S*. *epidermidis* ATCC 14990, *C*. *albicans* ATCC 90028 and *D. congolensis* ATCC 14637, resulting of CAPP application according to the invention to the research samples in contrast to control samples that have not been treated with this discharge. The experiment was performed in three biological repeats, each of them included two technical replicates.

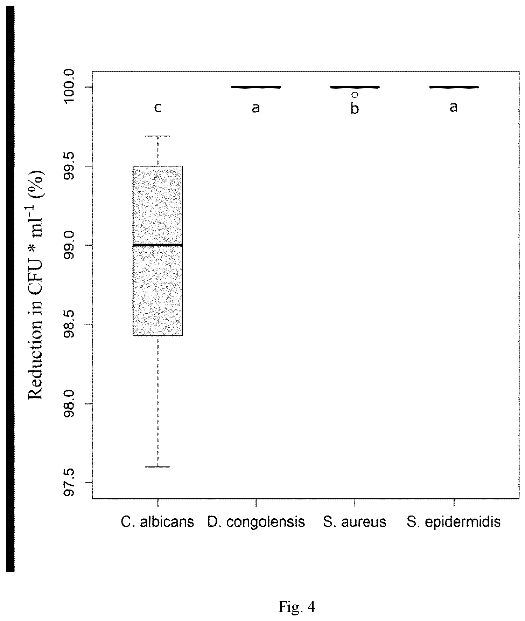

- Figure 4 shows eradication, as a result of exposure to CAPP generated in a plasma system with DBD in the form of a plasma jet, of microorganisms belonging to the species *S*. *aureus, S. epidermidis, C. albicans* and *D. congolensis,* causing wound infections, which had been previously applied to the porcine skin in a suspension of 0.5 McF optical density. The outcomes are depicted in a form of boxplot visualizing the median and individual data distribution quartiles of the observed percentage reduction in the number of colony-forming units of *S*. *aureus* ATCC 25923, *S*. *epidermidis* ATCC 14990, *C*. *albicans* ATCC 90028 and *D. congolensis* ATCC 14637, resulting of CAPP application according to the invention to the research samples in contrast to control samples that have not been treated with this discharge. The experiment was performed in three biological repeats, each of them included two technical replicates.

### Example 1.

*The method of eradication of microorganisms, responsible for skin infections in animals and humans, achieved by the use of CAPP and demonstrated by inactivation of cells of pathogenic microorganisms from a 0.5 McF optical density suspension placed on a flat surface*

The antimicrobial properties of CAPP, produced in a plasma system with DBD, generated in a form of a plasma jet, are proven by revealing the ability of the CAPP obtained in this way to eradicate from a flat surface the cells of the following pathogenic microorganisms responsible for skin infections in animals and humans: *S*. *aureus* ATCC 25923, *S*. *epidermidis* ATCC 14990, *C*. *albicans* ATCC 90028 and *D. congolensis* ATCC 14637, which had been applied to a polystyrene surface in suspension of 0.5 McF optical density.

The tested strains of microorganisms, *i.e. S. aureus* ATCC 25923, *S*. *epidermidis* ATCC 14990, *C*. *albicans* ATCC 90028 and *D. congolensis* ATCC 14637, are available in the bacterial collection of pathogens of the Intercollegiate Faculty of Biotechnology of the University of Gdańsk and the Medical University of Gdańsk (IFB UG & MUG) and kept at -80 °C in a form of frozen cultures. In order to perform experiments using a single colony of a stated microorganism, the pathogenic strains listed above are plated by applying the streaking technique on solid media. For inoculation of *S*. *aureus* ATCC 25923, *S*. *epidermidis* ATCC 14990 and *D. congolensis* ATCC 14637 Brain Heart Infusion-Agar (BHI-A) plates are used, while *C*. *albicans* ATCC 90028 yeasts are inoculated onto plates containing yeast extract, peptone and glucose solidified with agar (YPG-A). The inoculated solid media are incubated for 24 h at 37 °C for S. *aureus* ATCC 25923, *S*. *epidermidis* ATCC 14990 and *C*. *albicans* ATCC 90028 and for 72 h also at 37 °C, but in an atmosphere supplemented with 5% CO₂, for *D. congolensis* ATCC 14637. Afterwards, a single colony of the microorganism is picked using a sterile loop and inoculated into a liquid medium. *S*. *aureus* ATCC 25923, *S. epidermidis* ATCC 14990 and *D. congolensis* ATCC 14637 are grown in Brain Heart Infusion (BHI), while *C*. *albicans* ATCC 90028 is cultured in a liquid medium with yeast extract, peptone and glucose (YPG). The inoculated liquid media are incubated at 37 °C for 12 h with 140 rpm shaking. The so-obtained overnight cultures are centrifuged at 6500 rpm for 4 mins, the supernatant is removed and the pellet of microbial cells is resuspended in 1 ml of sterile Ringer's solution. The suspension of microorganisms in Ringer's solution is then subjected to another centrifugation at 6500 rpm for 4 mins, and the resultant supernatant is removed. The so-prepared pellet of microbial cells is resuspended in 1 ml of Ringer's solution. After the next washing of the pellet of microbial cells with Ringer's solution that is carried out as described above, the cells from the so-obtained pellet are resuspended in 1 ml of Ringer's solution. This microbial suspension is added to 10 ml of Ringer's solution placed in a glass tube of 15 ml capacity, in such a volume that, based on densitometric measurements using a DEN-1B densitometer (BioSan, Latvia), the 0.5 McF optical density of the microbial cell suspension is reached. The above-described activities are carried out for each tested pathogenic strain, *i.e. S. aureus* ATCC 25923, S. *epidermidis* ATCC 14990, *C*. *albicans* ATCC 90028 and *D. congolensis* ATCC 14637.

Into flat-bottomed 24-well microplates made of polystyrene, 500 µl of a cell suspension of the pathogenic microorganism (*S*. *aureus* ATCC 25923, *S*. *epidermidis* ATCC 14990, *C*. *albicans* ATCC 90028 or *D. congolensis* ATCC 14637) exhibiting an optical density of 0.5 McF is poured. The so-prepared 24-well plates are incubated at room temperature for 30 mins to allow the cells of the introduced microorganism to adhere to the bottom of the well. From above the cells absorbed onto polystyrene, the culture medium residuals are removed using an automatic pipette. The cells of the microorganisms present on the surface of the bottom of the well are subjected to a 1-minute exposure to CAPP, generated as a result of initiating DBD discharges in the reaction-discharge system (Figure 1) in a form of a plasma jet using the following parameters: flow rate of the discharge gas, *i.e.* helium: 5.2 L min⁻¹, duty cycle: 70%, frequency of modulation: 70%, plasma cone temperature = 37 °C. Post the CAPP action, 200 µl of sterile Ringer's solution is added to each well and shaken at 1000 rpm for 10 mins to detach the microbial cells that survived plasma treatment from the bottom of the wells. The next step is to prepare serial dilutions to 10⁻³ of the microbial cell suspension obtained in this way, in order to inoculate 100 µl of the so-obtained serial dilutions onto solid media (BHI-A: *S*. *aureus* ATCC 25923, *S*. *epidermidis* ATCC 14990 and *D. congolensis* ATCC 14637; YPG-A: *C. albicans* ATCC 90028) at the next stage of the experiment. After incubation at 37°C for 24 h in terms of *S. aureus* ATCC 25923, *S*. *epidermidis* ATCC 14990, *C*. *albicans* ATCC 90028 and 72 h in 5% CO₂ concerning *D. congolensis* ATCC 14637, calculation of the colony-forming units grown on the previously inoculated agar plates is performed. The number of colony-forming units of the CAPP-treated test samples is compared to the number of colony-forming units of the non-DBD-treated controls. The experiment is conducted on the strains *S*. *aureus* ATCC 25923, *S*. *epidermidis* ATCC 14990, *C*. *albicans* ATCC 90028 and *D. congolensis* ATCC 14637, introduced into 24-well plates within 0.5 McF optical density suspensions, in three biological replicates, including two technical repetitions each.

The effectiveness of the described method of eradication of microorganisms responsible for skin infections in animals and humans by the direct action of cold atmospheric plasma is assessed by determination of the percentage and logarithm of reduction in the number of colony-forming units of *S*. *aureus* ATCC 25923, *S*. *epidermidis* ATCC 14990, *C*. *albicans* ATCC 90028 and *D. congolensis* ATCC 14637, resulting from the action of CAPP generated in the DBD plasma system in a form of a plasma jet in terms of research samples in contrast to control samples not treated with this discharge.

The effectiveness of the reported method of eradicating microorganisms responsible for skin infections in animals and humans by direct CAPP exposure of microbial suspension of 0.5 McF optical density placed on a flat surface is shown in Fig. 2:
- Antimicrobial properties of CAPP, generated in a plasma system as a result of DBD initiation in a form of a plasma jet, towards microorganisms responsible for skin infections in animals and humans, which were applied to a flat surface in a suspension at 0.5 McF optical density (Fig. 2). The system for generating CAPP *via* DBD consists of a head of cold atmospheric plasma through which the gas flows and a high-frequency pulse generator as shown in Figure 1. The reaction-discharge system comprises a radio-frequency generator, modulated by pulses, connected to: a) a quartz capillary containing tungsten electrodes placed outside the quartz tube b) a cavity (head) made of epoxy resin c) a filling of the cavity made of silica and an insulator. As a result of the conducted tests, it was shown that 1 min of DBD action led to a reduction in the number of viable cells of the tested pathogenic microorganisms in the average range 99.97475-100%. Referring to log reduction, the applied discharge according to the invention resulted in a reduction in the number of viable microbial cells that exceeded an average 3.598 logs. Figure 2 depicts (box-plot chart, showing the median and the quartiles of data distribution. The circles correspond to outliers) the percentages of reduction in the number of CFUs of S. *aureus* ATCC 25923, *S*. *epidermidis* ATCC 14990, *C*. *albicans* ATCC 90028 and *D. congolensis* ATCC 14637, which had been previously applied in a form of 0.5 McF optical density suspensions to a flat surface, having in mind that the above-mentioned percentages of reduction in the number of viable cells of microorganisms responsible for skin infections were the outcomes of the exposure of the inoculated flat surface to the generated CAPP and the research samples were juxtaposed to the non-plasma-treated control groups. Data differing in a statistically significant manner (Kruskal-Wallis test, *p* < 0.05) have been marked with a different letter.

### Example 2.

*The method of eradication of microorganisms, responsible for skin infections in animals and humans, achieved by the use of CAPP and demonstrated by inactivation of cells of pathogenic microorganisms from a 0.05 McF optical density suspension placed on a flat surface*

The antimicrobial properties of CAPP, produced in a plasma system with DBD, generated in a form of a plasma jet, are proven by revealing the ability of the CAPP obtained in this way to eradicate from a flat surface the cells of the following pathogenic microorganisms responsible for skin infections in animals and humans: *S. aureus* ATCC 25923, *S*. *epidermidis* ATCC 14990, *C*. *albicans* ATCC 90028 and *D. congolensis* ATCC 14637, which had been applied to a polystyrene surface in suspension of 0.05 McF optical density.

The tested strains of microorganisms, *i.e. S. aureus* ATCC 25923, *S*. *epidermidis* ATCC 14990, *C*. *albicans* ATCC 90028 and *D. congolensis* ATCC 14637, are available in the bacterial collection of pathogens of the Intercollegiate Faculty of Biotechnology of the University of Gdańsk and the Medical University of Gdańsk (IFB UG & MUG) and kept at -80 °C in a form of frozen cultures. In order to perform experiments using a single colony of a stated microorganism, the pathogenic strains listed above are plated by applying the streaking technique on solid media. For inoculation of *S*. *aureus* ATCC 25923, *S*. *epidermidis* ATCC 14990 and *D. congolensis* ATCC 14637 Brain Heart Infusion-Agar (BHI-A) plates are used, while *C*. *albicans* ATCC 90028 yeasts are inoculated onto plates containing yeast extract, peptone and glucose solidified with agar (YPG-A). The inoculated solid media are incubated for 24 h at 37 °C for *S*. *aureus* ATCC 25923, *S*. *epidermidis* ATCC 14990 and *C*. *albicans* ATCC 90028 and for 72 h also at 37 °C, but in an atmosphere supplemented with 5% CO₂, for *D. congolensis* ATCC 14637. Afterwards, a single colony of the microorganism is picked using a sterile loop and inoculated into a liquid medium. *S*. *aureus* ATCC 25923, *S. epidermidis* ATCC 14990 and *D. congolensis* ATCC 14637 are grown in Brain Heart Infusion (BHI), while *C*. *albicans* ATCC 90028 is cultured in a liquid medium with yeast extract, peptone and glucose (YPG). The inoculated liquid media are incubated at 37 °C for 12 h with 140 rpm shaking. The so-obtained overnight cultures are centrifuged at 6500 rpm for 4 min, the supernatant is removed and the pellet of microbial cells is resuspended in 1 ml of sterile Ringer's solution. The suspension of microorganisms in Ringer's solution is then subjected to another centrifugation at 6500 rpm for 4 min, and the resultant supernatant is removed. The so-prepared pellet of microbial cells is resuspended in 1 ml of Ringer's solution. After the next washing of the pellet of microbial cells with Ringer's solution that is carried out as described above, the cells from the so-obtained pellet are resuspended in 1 ml of Ringer's solution. This microbial suspension is added to 10 ml of Ringer's solution placed in a glass tube of 15 ml capacity, in such a volume that, based on densitometric measurements using a DEN-1B densitometer (BioSan, Latvia), the 0.5 McF optical density of the microbial cell suspension is reached. The 0.05 McF optical density of the microbial suspension applied in this experiment is obtained by diluting the previously prepared 0.5 McF optical density cell suspension with sterile Ringer's solution at a 1:9 volume ratio. The above-described activities are carried out for each tested pathogenic strain, *i.e. S. aureus* ATCC 25923, *S*. *epidermidis* ATCC 14990, *C*. *albicans* ATCC 90028 and *D. congolensis* ATCC 14637.

Into flat-bottomed 24-well microplates made of polystyrene, 500 µl of a cell suspension of the pathogenic microorganism (*S*. *aureus* ATCC 25923, *S*. *epidermidis* ATCC 14990, *C*. *albicans* ATCC 90028 or *D. congolensis* ATCC 14637) exhibiting an optical density of 0.05 McF is poured. The so-prepared 24-well plates are incubated at room temperature for 30 min to allow the cells of the introduced microorganism to adhere to the bottom of the well. From above the cells absorbed into the polystyrene, the culture medium residuals are removed using an automatic pipette. The cells of the microorganisms present on the surface of the bottom of the well are subjected to a 1-minute exposure to CAPP, generated as a result of initiating DBD discharges in the reaction-discharge system (Figure 1) in a form of a plasma jet using the following parameters: flow rate of the discharge gas, *i.e.* helium: 5.2 L min⁻¹, current fill value: 70%, frequency value modulating this current: 70%, plasma cone temperature = 37 °C. Post the CAPP action, 200 µl of sterile Ringer's solution is added to each well and shaken at 1000 rpm for 10 min to detach the microbial cells that survived plasma treatment from the bottom of the wells. The next step is to prepare serial dilutions to 10⁻³ of the microbial cell suspension obtained in this way, in order to inoculate 100 µl of the so-obtained serial dilutions onto solid media (BHI-A: *S*. *aureus* ATCC 25923, *S. epidermidis* ATCC 14990 and *D. congolensis* ATCC 14637; YPG-A: *C. albicans* ATCC 90028) at the next stage of the experiment. After incubation at 37°C for 24 h in terms of *S*. *aureus* ATCC 25923, *S*. *epidermidis* ATCC 14990, *C*. *albicans* ATCC 90028 and 72 h in 5% CO₂ concerning *D. congolensis* ATCC 14637, calculation of the colony-forming units grown on the previously inoculated agar plates is performed. The number of colony-forming units in the CAPP-treated test samples is compared to the number of colony-forming units in the non-DBD-treated controls. The experiment is conducted on the strains *S*. *aureus* ATCC 25923, *S*. *epidermidis* ATCC 14990, *C*. *albicans* ATCC 90028 and *D. congolensis* ATCC 14637, introduced into 24-well plates within 0.05 McF optical density suspensions, in three biological replicates, including two technical repetitions each.

The effectiveness of the described method of eradication of microorganisms responsible for skin infections in animals and humans by the direct action of cold atmospheric plasma is assessed by determination of the percentage and logarithm of reduction in the number of colony-forming units of *S*. *aureus* ATCC 25923, *S*. *epidermidis* ATCC 14990, *C*. *albicans* ATCC 90028 and *D. congolensis* ATCC 14637, resulting from the action of CAPP generated in the DBD plasma system in a form of a plasma jet in terms of research samples in contrast to control samples not treated with this discharge.

The effectiveness of the reported method of eradicating microorganisms responsible for skin infections in animals and humans by direct CAPP exposure of microbial suspension of 0.05 McF optical density placed on a flat surface is shown in Fig. 3:
- Antimicrobial properties of CAPP, generated in a plasma system as a result of DBD initiation in a form of a plasma jet, towards microorganisms responsible for skin infections in animals and humans, which were applied to a flat surface in a suspension at 0.05 McF optical density (Fig. 3). As a result of the conducted tests, it was shown that 1 min of DBD action produced in the reaction-discharge system (Fig. 1) led to a reduction in the number of viable cells of the tested pathogenic microorganisms in the average range 99.9968-100%. Referring to log reduction, the applied discharge according to the invention resulted in a reduction in the number of viable microbial cells that exceeded on average 4.494 logs. The figure depicts (box-plot chart, showing the median and the quartiles of data distribution. The circles correspond to outliers) the percentages of reduction in the number of colonies of *S*. *aureus* ATCC 25923, *S*. *epidermidis* ATCC 14990, *C*. *albicans* ATCC 90028 and *D. congolensis* ATCC 14637, which had been previously applied in a form of 0.05 McF optical density suspensions to a flat surface, having in mind that the above-mentioned percentages of reduction in the number of viable cells of microorganisms responsible for skin infections were the outcomes of the exposure of the inoculated flat surface to the generated CAPP and the research samples were juxtaposed to the non-plasma-treated control groups. Data differing in a statistically significant manner (Kruskal-Wallis test, *p* < 0.05) have been marked with a different letter.

### Example 3.

*The method of eradication of microorganisms, responsible for skin infections in animals and humans, achieved by the use of CAPP and demonstrated by inactivation of cells of pathogenic microorganisms from a surface of porcine skin*

The antimicrobial properties of CAPP, produced in a plasma system with DBD, generated in a form of a plasma jet, are proven by revealing the ability of the CAPP obtained in this way to eradicate from a surface of porcine skin the cells of the following pathogenic microorganisms responsible for skin infections in animals and humans: *S*. *aureus* ATCC 25923, *S*. *epidermidis* ATCC 14990, *C*. *albicans* ATCC 90028 and *D. congolensis* ATCC 14637.

Fresh porcine skin, acquired from a local butchery, is processed and sterilized as follows. At first, fragments of connective and muscle tissue are removed from beneath the porcine skin. The material purified in this way is cut into pieces of approx. 1 cm², which are immersed for 10 mins in 70% ethanol in order to perform initial sterilization of the animal-derived material. After chemical sterilization, fragments of porcine skin are placed on the surface of sterile 96 mm in diameter Petri dishes and subjected to 20-mins physical sterilization using UV irradiation. The skin fragments sterilized in this way are placed on the surface of sterile Petri dishes, secured with parafilm, and subsequently kept at -20 °C for subsequent analyses.

The cell suspensions of pathogenic microorganisms of optical density 0.5 McF, needed for artificial infection of the previously sterilized porcine skin, are prepared for the following microorganisms *S*. *aureus* ATCC 25923, *S*. *epidermidis* ATCC 14990, *C*. *albicans* ATCC 90028 and *D. congolensis* ATCC 14637, which were acquired from the bacterial collection of pathogens of the Intercollegiate Faculty of Biotechnology of the University of Gdańsk and the Medical University of Gdańsk (IFB UG & MUG), in which they are kept at -80 °C in 40% (v/v) glycerol in the form of frozen cultures. In order to perform further experiments using a single colony of a stated microorganism, the listed-above pathogenic strains are plated by applying the streaking technique on solid media. Concerning *S*. *aureus* ATCC 25923, *S*. *epidermidis* ATCC 14990 and *D. congolensis* ATCC 14637 BHI-A plates are used for inoculation, while *C*. *albicans* ATCC 90028 yeasts are inoculated onto YPG-A plates. The inoculated solid media are incubated for 24 h at 37 °C for *S*. *aureus* ATCC 25923, *S*. *epidermidis* ATCC 14990 and *C*. *albicans* ATCC 90028 and for 72 h also at 37 °C, but in an atmosphere supplemented with 5% CO₂, for *D. congolensis* ATCC 14637. Afterwards, a single colony of the microorganism is picked using a sterile loop and inoculated into a liquid medium. *S*. *aureus* ATCC 25923, *S*. *epidermidis* ATCC 14990 and *D. congolensis* ATCC 14637 are grown in BHI broth, while *C*. *albicans* ATCC 90028 is cultured in a liquid YPG medium. The inoculated liquid media are incubated at 37 °C for 12 h with 140 rpm shaking. The so-obtained overnight cultures are centrifuged at 6500 rpm for 4 mins, the supernatant is removed and the pellet of microbial cells is resuspended in 1 ml of sterile Ringer's solution. The suspension of microorganisms in Ringer's solution is then subjected to another centrifugation at 6500 rpm for 4 mins, and the resultant supernatant is removed. The so-prepared pellet of microbial cells is resuspended in 1 ml of Ringer's solution. After the last washing of the pellet of microbial cells with Ringer's solution that is carried out as described above, the cells from the so-obtained pellet are resuspended in 1 ml of Ringer's solution. This microbial suspension is added to 10 ml of Ringer's solution placed in a glass tube of 15 ml capacity, in such a volume that, based on densitometric measurements using a DEN-1B densitometer (BioSan, Latvia), the 0.5 McF optical density of the microbial cell suspension is reached. The above-described activities are carried out for each tested pathogenic strain, *i.e. S. aureus* ATCC 25923, *S*. *epidermidis* ATCC 14990, *C*. *albicans* ATCC 90028 and *D. congolensis* ATCC 14637.

Artificial infection of the sterilized as described above porcine skin with the previously prepared 0.5 McF optical density suspensions of microorganisms is carried out as follows. 1 ml of 0.5 McF optical density suspension of the cells of microorganisms responsible for skin infections in animals and humans, *i.e. S. aureus* ATCC 25923, S. *epidermidis* ATCC 14990, *C*. *albicans* ATCC 90028 or *D. congolensis* ATCC 14637, is introduced into a sterile 1.5 ml Eppendorf tube. The microbial suspension is then centrifuged at 6500 rpm for 7 mins. The supernatant is discarded by inverting the tube, and the microbial cells remaining at the bottom of the tube are resuspended by pipetting in the remaining residuals of Ringer's solution (approx. 20 µl). Subsequently, the Petri dish, containing sterile fragments of porcine skin, is transferred from the freezer (-20 °C) to a biosafety cabinet in order to thaw the tissue. The thawing process is carried out for 20 minutes and the porcine skin fragments are additionally exposed to UV irradiation during this period. The next step of the work is the transfer of surface-sterilized and thawed porcine skin fragments to the surface of the HEPES medium solidified with 1.5% agar and this medium had been poured before on 96 mm in diameter Petri dishes. The suspension containing microorganisms is then spread evenly over the surface of the porcine skin using an automatic pipette. The artificially-inoculated porcine skin is then placed in an incubator set at 37 °C for 24 hours to allow for colonization of the material by the introduced microorganisms. After this time, the colonized porcine skin is exposed directly to CAPP for 2 mins. The system for generating CAPP *via* DBD consists of a cold atmospheric plasma head through which the gas flows and a high-frequency pulse generator (Fig. 1). After an interval of 2 mins, the animal-derived material is exposed a second time to CAPP for another 2 mins. The applied plasma, generated in two cycles, was initiated as a result of DBD generation in a form of a plasma jet using the following parameters: discharge gas flow rate, *i.e.* helium: 4.0 L min⁻¹, duty cycle: 70%, frequency of modulation: 70%, plasma cone temperature = 37 °C.

The following procedure is used to calculate the number of microbial cells that survived treatment with DBD discharge, which was generated in a form of a plasma jet. A sterile swab stick moistened in Ringer's solution is used to swab DBD-treated porcine skin, which was previously artificially inoculated with the cells of microorganisms responsible for skin infections in animals and humans (*S. aureus* ATCC 25923, *S*. *epidermidis* ATCC 14990, *C*. *albicans* ATCC 90028 or *D. congolensis* ATCC 14637). The swab is taken by pulling the swab stick 5 times to the left and to the right on the surface of the central part of a 1 cm² fragment of porcine skin. The swab stick with the collected cells is placed in 300 µl of Ringer's solution in a 2 ml volume Eppendorf tube. Next, the swab stick is aseptically cut to allow closure of the cap of the Eppendorf tube. The Eppendorf tube is subjected to vigorous shaking for 1 min on a vortex followed by centrifugation at 6500 rpm for 8 min. The supernatant is then discarded and the remaining at the bottom of the tube microbial cells are resuspended in 100 µl of Ringer's solution. From the cell suspension obtained in this manner, serial dilutions in Ringer's solution up to 10⁻⁸ (volume ratio 1:9) are prepared and the sample dilution process is carried out in 96-well microplates. Subsequently, 20 µl of the previously prepared serial dilutions are absorbed with an 8-channel pipette and streaked in a form of stripes by slowly releasing the microorganisms-containing solutions from the automatic pipette onto the surface of the solid medium along its entire length. *S*. *aureus* ATCC 25923, *S*. *epidermidis* ATCC 14990 and *D. congolensis* ATCC 14637 are inoculated onto BHI-A plates, while *C*. *albicans* ATCC 90028 is streaked onto YPG-A plates. After incubation of the inoculated plates at 37°C for 24 h in terms of *S*. *aureus* ATCC 25923, *S. epidermidis* ATCC 14990 and *C*. *albicans* ATCC 90028 and 72 h for *D. congolensis* ATCC 14637 also at 37°C but in an atmosphere of 5% CO₂, the colony-forming units that grew on the inoculated agar plates are counted. The number of colony-forming units in the CAPP-treated test samples is compared to the number of colony-forming units in the non-DBD-treated controls. The experiment is performed for the strains *S*. *aureus* ATCC 25923, *S*. *epidermidis* ATCC 14990, *C*. *albicans* ATCC 90028 and *D. congolensis* ATCC 14637, in three biological replicates, each including two technical replicates.

The effectiveness of the described method of eradication of microorganisms responsible for skin infections in animals and humans by the direct action of cold atmospheric plasma is assessed by determination of the percentage and logarithm of reduction in the number of colony-forming units of *S*. *aureus* ATCC 25923, *S*. *epidermidis* ATCC 14990, *C*. *albicans* ATCC 90028 and *D. congolensis* ATCC 14637, resulting from the action of CAPP generated in the DBD plasma system in a form of a plasma jet in terms of research samples in contrast to the control samples not treated with this discharge.

The effectiveness of the reported method of eradicating microorganisms responsible for skin infections in animals and humans by direct CAPP exposure of microorganisms artificially inoculated on the surface of porcine skin is shown in Fig.4:
- Antimicrobial properties of CAPP, generated in a plasma system as a result of DBD initiation in a form of a plasma jet, towards microorganisms responsible for skin infections in animals and humans, which were artificially inoculated onto the surface of porcine skin (Fig. 4). As a result of the conducted tests, it was shown that two expositions for 2 mins to the action of DBD generated in the reaction-discharge system (Fig. 1) led to a reduction in the number of viable cells of the tested pathogenic microorganisms in the average range 98.8698-100%. Referring to log reduction, the applied discharge according to the invention resulted in a reduction in the number of viable microbial cells that exceeded an average 1.94683 logs. The figure depicts (box-plot chart, showing the median and the quartiles of data distribution. The circles correspond to outliers) the percentages of reduction in the number of colonies of *S*. *aureus* ATCC 25923, *S*. *epidermidis* ATCC 14990, *C*. *albicans* ATCC 90028 and *D. congolensis* ATCC 14637, which had previously colonized the surface of porcine skin, having in mind that the above-mentioned percentages of reduction in the number of viable cells of microorganisms responsible for skin infections were the outcomes of the exposure of the mentioned surface of the porcine skin to the generated CAPP and the research samples were juxtaposed to the non-plasma-treated control groups. Data differing in a statistically significant manner (Kruskal-Wallis test, *p* < 0.05) have been marked with a different letter.

## Claims

1. Method of eradicating pathogenic microorganisms from non-body flat surfaces **characterized in that** flat surfaces are directly exposed to cold atmospheric pressure plasma (CAPP), generated as barrier discharges (DBD) in a form of a plasma jet, **characterized in that**, atmospheric plasma is generated using an alternating current with a duty cycle of 70% and a frequency of modulation amplitude of 70%, and the eradication of microbes is carried out using helium as a discharge gas to generate the CAPP, which is applied at a flow rate of 2-10 L/min,
wherein the reaction-discharge system in which the plasma is generated consists of a current generator connected to tungsten electrodes located on: a quartz capillary placed in an epoxy resin cavity tip filled with silica and an insulator.

2. The method according to claim 1, wherein the flat surface from which the microorganisms are eradicated is treated once or twice with a DBD discharge.

3. The method according to claim 1, wherein the flat surface from which the microorganisms are eradicated is treated with a DBD discharge, and such exposure lasts from 10 seconds to 10 mins, most preferably 2 minutes.

4. The method according to claim 1, wherein the distance between the exposed flat surface and the CAPP cone is 2 mm-10 cm, most preferably 3 cm.

5. The method according to claims 1-3, wherein the DBD discharge inactivates the cells of pathogenic microorganisms, in particular microorganisms from the species *S*. *aureus, S. epidermidis, C. albicans* and *D*. *congolensis.*

6. The method according to claim 1, wherein the DBD discharge is applied to flat surfaces.

7. Reaction-discharge system for eradication of pathogenic microorganisms from flat surfaces, comprising a source for generating plasma having a plasma generator with an electrode; a source of electrical power connected to the plasma generator and a source of a gas, wherein cold atmospheric plasma (CAPP) is generated as a result of barrier discharges (DBD), in a form of a plasma jet, and the system is based on a current generator connected to tungsten electrodes located on a quartz capillary placed in a cavity tip
**characterized in that** the cavity tip is made of epoxy resin filled with silica and an insulator. and in this reaction-discharge system helium is applied at a flow rate of 2-10 L/min as a discharge gas to produce CAPP, and the DBD discharge is generated using an alternating current of a duty cycle of 70% and a frequency of modulation amplitude of 70%.

## Patentansprüche

1. Verfahren zur Eradikation pathogener Mikroorganismen von flachen Oberflächen, **dadurch gekennzeichnet, dass** die Oberflächen direkt kaltem Atmosphärendruckplasma (KAP/ Engl.= Cold Atmospheric Pressure Plasma (CAPP)) ausgesetzt werden, das als Dielektrische Barriereentladungen (DBE/ Engl. = Dielectric Barrier Discharge (DBD)) in Form eines Plasmastrahls erzeugt wird,
**dadurch gekennzeichnet, dass** atmosphärisches Plasma unter Verwendung eines Wechselstroms mit einem Tastverhältnis von 70% und einer Modulationsamplitude von 70% erzeugt wird, und die Beseitigung der Mikroben unter Verwendung von Helium als Entladungsgas zur Erzeugung des KAPs, das mit einer Durchflussrate von 2-10 L/min aufgebracht wird, wobei das Reaktions-Entladungssystem, in dem das Plasma erzeugt wird, aus einem Stromgenerator besteht, der mit Wolframelektroden verbunden ist, die sich an der folgenden Stelle befinden: einer Quarzkapillare, die in einer mit Siliziumdioxid und einem Isolator gefüllten Epoxidharz-Hohlkehle angeordnet ist.

2. Verfahren nach Anspruch 1, wobei die flache Oberfläche, von der die Mikroorganismen beseitigt werden, ein- oder zweimal mit einer DBE behandelt wird.

3. Verfahren nach Anspruch 1, wobei die flache Oberfläche, von der die Mikroorganismen beseitigt werden, 10 Sekunden bis 10 Minuten, jedoch vorzugsweise 2 Minuten mit einer DBE behandelt wird

4. Verfahren nach Anspruch 1, wobei der Abstand zwischen der Oberfläche und dem KAP-Kegel 2 mm bis 10 cm, jedoch vorzugsweise 3 cm beträgt.

5. Verfahren nach Ansprüchen 1-3, wobei die DBE die Zellen pathogener Mikroorganismen, insbesondere Mikroorganismen der Arten *S. aureus, S. epidermidis, C. albicans und D. congolensis*, inaktiviert.

6. Verfahren nach Anspruch 1, wobei die DBE auf flache Oberflächen angewendet wird.

7. Reaktionsentladungssystem zur Eradikation pathogener Mikroorganismen auf flachen Oberflächen, das eine Quelle zur Erzeugung von Plasma mit einem Plasmagenerator mit einer Elektrode; eine mit dem Plasmagenerator verbundene Stromquelle und eine Gasquelle umfasst, wobei das KAP als Ergebnis von DBE in Form eines Plasmastrahls erzeugt wird, wobei das System auf einem Stromgenerator basiert, der mit Wolframelektroden verbunden ist, die sich auf einer Quarzkapillare befinden, die in einer Hohlkehle angeordnet ist, **dadurch gekennzeichnet, dass** diese Hohlkehle aus Epoxidharz besteht und mit Siliziumdioxid und einem Isolator gefüllt ist, und dass in diesem Reaktions-Entladungssystem Helium mit einer Durchflussrate von 2-10 I/min als Entladungsgas zur Erzeugung vom KAP verwendet wird und die DBE unter Verwendung eines Wechselstroms mit einem Tastverhältnis von 70% und einer Modulationsamplitude von 70% erzeugt wird

## Revendications

1. Méthode d'éradication de micro-organismes pathogènes de surfaces planes non corporelles caractérisée dans ces surfaces planes qui sont directement exposées à un plasma atmosphérique froid sous pression (PAFSP), généré en tant que décharges à barrière diélectrique (DBD) sous la forme d'un jet de plasma, où le plasma atmosphérique est généré en utilisant un courant alternatif avec un cycle de service de 70 % et une fréquence de modulation d'amplitude de 70 %, et l'éradication des microbes est réalisée en utilisant l'hélium comme gaz de décharge pour générer le PAFSP, ce dernier est appliqué à un débit de 2-10 L/min,
où le système réaction-décharge dans lequel le plasma est généré se compose d'un générateur de courant connecté à des électrodes en tungstène situées sur : un capillaire en quartz placée dans l'embout d'une cavité en résine époxy remplie de silice et d'un isolant.

2. La méthode selon la revendication 1, dans laquelle la surface plane dont les micro-organismes sont éradiqués est traitée une ou deux fois par une décharge DBD.

3. La méthode selon la revendication 1, dans laquelle la surface plane dont les micro-organismes sont éradiqués est traitée par une décharge DBD, et une telle exposition dure de 10 secondes à 10 minutes, de préférence 2 minutes.

4. La méthode selon la revendication 1, dans laquelle la distance entre la surface plane exposée et le cône du PAFSP est de 2 mm à 10 cm, de préférence 3 cm.

5. La méthode selon les revendications 1-3, dans laquelle la décharge DBD inactive les cellules de micro-organismes pathogènes, en particulier des micro-organismes des espèces *S*. *aureus, S. epidermidis, C. albicans et D. congolensis.*

6. La méthode selon la revendication 1, dans laquelle la décharge DBD est appliquée à des surfaces planes.

7. Le système réaction-décharge pour l'éradication de micro-organismes pathogènes de surfaces planes, comprenant une source pour générer le plasma comportant un générateur de plasma avec une électrode ; une source d'alimentation électrique connectée au générateur de plasma et une source de gaz où le plasma atmosphérique froid (PAFSP) est généré à la suite de décharges à barrière diélectrique (DBD), sous la forme d'un jet de plasma, et le système est basé sur un générateur de courant connecté à des électrodes en tungstène situées sur un capillaire en quartz placée dans l'embout d'une cavité, **caractérisé en ce que** l'embout de la cavité est en résine époxy remplie de silice et d'un isolant et dans ce système réaction-décharge le hélium est appliqué à un débit de 2 - 10 L/min en tant que gaz de décharge pour produire le PAFSP et la décharge DBD est générée à l'aide d'un courant alternatif présentant un rapport cyclique de 70 % et une fréquence de modulation d'amplitude de 70 %.
